# EUROPEAN PATENT APPLICATION

(11) **EP 2 520 297 A1**
(43) Date of publication of application: **07.11.2012**
(21) Application number: 11290215.0
(22) Date of filing: 04.05.2011
(51) Int. Cl.: A61K 31/407, A61P 33/00, A61P 33/06, A01P 13/00, A01N 43/38

(54) **Rubreserine and its derivates with antiparasitic activity**

(71) Applicant: Commissariat à l'Énergie Atomique et aux Énergies Alternatives, 75015 Paris (FR); Centre National de la Recherche Scientifique, 75016 Paris (FR)
(72) Inventor: Camara, Djeneb, 38100 Grenoble (FR); Bisanz, Cordelia, 38220 Saint Barthelemy de Sechilienne (FR); Barette, Caroline, 38000 Grenoble (FR); Cesbron-Delauw, Marie-France, 38700 La Tronche (FR); Rébeillé, Fabrice, 38340 Voreppe (FR)
(74) Representative: Mena, Sandra

(57) **Abstract**

The invention relates to novel compounds responding to the following formula (I): 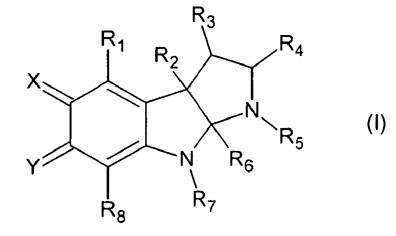 or a pharmaceutically acceptable salt thereof,
for use as medicaments, and in particular as medicaments for the prevention and/or the treatment of parasitic diseases caused by apicomplexans parasites. The invention also concerns pharmaceutical compositions containing such compounds of formula (I) as active principles. Finally, the present invention relates to the use of compounds of formula (I) as herbicides and/or algaecides and to herbicide and/or algaecide compositions containing such compounds.

## Description

The invention relates to novel compounds for use as medicaments, and in particular as medicaments for the prevention and/or the treatment of parasitic diseases caused by apicomplexans parasites. The invention also relates to pharmaceutical compositions containing such compounds of formula (I) as active principles. Finally, the present invention relates to the use of compounds of formula (I) as herbicides and/or algaecides and to herbicide and/or algaecide compositions containing such compounds.

The *Apicomplexa phylum* constitutes a particularly diverse group of unicellular protists. Besides, members of the Colpodellida sub-group, all apicomplexans are obligate intracellular parasites, infecting virtually all animals from mollusks to mammals. They were originally classified as protozoa (> 4000 species). Amongst them, the malaria parasite *Plasmodium sporozoa,* represents one of the most important threats for humankind, with 300 to 500 million estimated clinical cases and 1.5 to 3 million deaths each year, most of them African children. More benign, but widely spread is *Toxoplasma gondii,* a parasite causing congenital neurological birth defects and, along with *Cryptosporidium,* being the most reported opportunistic infection associated with immunosuppressive conditions, including AIDS. Other parasites in this phylum are of veterinary importance including pathogens of cattle and chicken, *Theileria* and *Eimeria.*

The *Apicomplexa phylum* contains several thousands of unicellular parasites among which:
- *Plasmodium falciparum,* the major malaria causative agent, as well as other parasites of the same genus (the three other *Plasmodium* species that cause human malaria: *Plasmodium vivax, Plasmodium ovale, Plasmodium malariae;* the *Plasmodium* species known to infect other vertebrates: *Plasmodium yoelii, Plasmodium berghei, Plasmodium chabaudi, Plasmodium vivax, Plasmodium gallinaceum, Plasmodium knowlesi, Plasmodium reichenowi,* etc.);
- *Babesia microti,* the causative agent of human babesiosis, as well as all other parasites that cause babiesosis in mammals (*i.e. Babesia divergens, Babesia bigemina, Babesia major, Babesia bovis* (bovine babesiosis), *Babesia canis, Babesia gibsoni* (canine babesiosis), *Babesia equi, Babesia caballi* (equine babesiosis), etc.);
- *Toxoplasma gondii,* the causative agent of toxoplasmosis in humans and other mammals;
- *Neospora caninum,* the causative agent of neosporosis;
- *Cryptosporidium hominis,* the causative agent of human cryptosporidiosis, as well as other parasites of the same genus (*Cryptosporidium parvum,* etc.);
- *Theileria annulata,* the causative agent of theileriosis, as well as other parasites of the same genus (*Theileria parva,* etc.)
- *Sarcocystis neurona, Eimeria tenella, Gregarina niphandrodes,* as well as any other parasites of the *Apicomplexa phylum* causing infectious diseases in metazoans.

All apicomplexans share a polarized cellular organization schema, with a basal pole and an apical pole. Membrane compartmentalization exhibits some specific features as compared to other eukaryotic cells such as those of yeasts or mammals. The cell is polarized and exhibits a set of subcellular structures called the apical complex that contains a hollow truncated cone, the conoid, and three types of vesicular secretory organelles, namely rhoptries, micronemes and dense granules. During host cell invasion, the apical pole is the first to enter the cell. Membrane compartments include those known to be connected through the endomembrane flow in all eukaryotic cells, *i*.*e*. the Golgi, endoplasmic reticulum, nuclear envelope and plasma membrane. A peripheral inner membrane complex is applied to the plasma membrane surface. Semi-autonomous organelles include a mitochondria surrounded by two membranes, and the apicoplast (absent in some genera such as *Cryptosporidium*) surrounded by four membranes.

The apicomplexan parasites are transmitted by different vectors in complex life cycles. For instance, and like all other malaria parasites of the *Plasmodium* genus, *Plasmodium falciparum* spreads by infecting successively two types of hosts: humans and female Anopheles mosquitoes. In humans, the parasites grow and multiply first in the liver cells and then in the red cells of the blood. In the blood, successive broods of parasites grow inside the red cells and destroy them, releasing daughter parasites ("merozoites") that continue the cycle by invading other red cells. The blood stage parasites are those that cause the symptoms of malaria. When certain forms of blood stage parasites ("gametocytes") are picked up by a female Anopheles mosquito during a blood meal, they start another, different cycle of growth and multiplication in the mosquito. After 10-18 days, the parasites are found (as "sporozoites") in the mosquito's salivary glands. When the Anopheles mosquito takes a blood meal on another human, the sporozoites are injected with the insect's saliva and start another human infection.

Chemotherapies are needed to fight against apicomplexan-caused diseases. These therapies generally differ with the type of parasite responsible of the infection.

In the case of malaria, this life-threatening disease affects half a billion humans in underdeveloped and developing countries. Its global heartland is Africa, with an appalling death toll of 1 to 2 million people every year (WHO World Malaria Report, Geneva, World Health Organization, WHO/UNICEF; 2005). Endemic malaria ranges from a permanent incidence in sub-Saharan and equatorial Africa, to a seasonal but recently escalating prevalence in Southern Africa Grover-Kopec et al., Malar. J. 2006, 5:38). Malaria was eradicated from temperate regions following concerted preventative sanitary actions and after important insecticide campaigns and systematic treatments with available drugs, *i.e.* quinine and chloroquine. The prophylactic programs of the 1950's and 1960's, essentially based on insecticide and drug treatments, failed to control malaria in subtropical areas. Resistance to chloroquine spread rapidly. Subsequent attempts to achieve progress in malaria prophylaxis have been characterized by the failure of vaccine development, withdrawal of some insecticides because of toxicity and negative environmental impact, the alarming spread of mosquito resistance to insecticides and of resistance of *Plasmodium* to the very few drugs that have been developed.

In an effort to improve the success rate of drug development, several approaches are being investigated. Most of the strategies are based on re-design of existing drugs, for example artemisinin, an antiplasmodial molecule from *Artemisia annua* comprising an endoperoxide moiety, and derivatives thereof such as Artesunate, which can be produced efficiently and cheaply (Renslo A. R. et al., Nature Chem. Biol., 2006, 2, 701-710).

Others choose to only keep the endoperoxyde moiety of artemisinin, leading to the discovery of synthetic RBx 11160 (OZ277) which mimics the chemical and biological properties of this molecule and is already in clinical trials as antimalaria drug used with piperaquine (Vennerstrom, J.L. et al., Nature, 2004, 430, 900-904).

The novel use of combination of older drugs is also under investigation: Lapdap^{®} (Chloroproguanil-dapsone) or Lapdap^{®}-artesunate are based on combinations of chloroproguanil (CPG) and dapsone (DDS) and was launched in 2003 as an effective and cheap antimalarial (Mutabingwa T. et al., Lancet, 2001, 358, 1218-1223).

Another well known drug is the quinolines family. Work on this old drug eventually succeeded to introduce antiplasmodial compounds such as quinoline and isoquine derivatives (Madrid, P.B. et al., J. J. Med. Chem. 2007, 50, 889-896). Recently astemizole, a known antihistaminic drug was shown to have a similar activity toward *Plasmodium* than artemisinin and other derivatives (Chong, C.R., et al., Nat. Chem. Biol., 2006, 2, 415-416).

Current efforts focus therefore on chemotherapy using multiple therapies including artemisinin. However, the scientific community is worried that plans for the extensive use of artemisinin might be ruined by emergence of the parasitic resistance it will almost certainly trigger, sooner or later. Given the small number of available drugs and the resistance they have already induced, discovery of new targets and of new drugs remains a key priority.

In the case of toxoplasmosis, *Toxoplasma gondii* has a broad spectrum of hosts and can virtually infect any mammal. It is well established that the final hosts are cats where sexual reproduction occurs. Transmission to humans occurs most often by infected food, or by direct or indirect contacts with cats. Severity of toxoplasmosis is very variable. In healthy human adults, infection is usually not visible besides serum analyses. Most healthy people do not require any treatment. In infected patients, the parasite remains as cysts that can reactivate a toxoplasmosis in case of immune deficiencies. Treatments are critically needed in the case of pregnant women and babies, people with HIV/AIDS and patient treated for cancer by chemotherapies. Most widely used treatments include pyrimethamine and sulfadiazine.

Because these drugs can have serious side effects for both women and babies, they are normally not used during pregnancy. Drug treatment can lessen the severity of congenital toxoplasmosis, but it will not undo any damage that has already occurred. Pyrimethamine is an anti-malarial medication also used to treat toxoplasmosis, and acts as a folic acid antagonist. It may prevent the body from absorbing the important B vitamin folate (folic acid, vitamin B-9), especially when taking high doses over a long period of time. Other potential side effects of pyrimethamine include bone marrow suppression and liver toxicity. Sulfadiazine is an antibiotic used in combination with pyrimethamine to treat toxoplasmosis. Possible side effects include nausea, vomiting and diarrhea. Pyrimethamine can also be supplied in conjunction with clindamycin, an antibiotic that can sometimes cause severe diarrhea. Patients suffering from AIDS need to take these medications for life, although in some cases, toxoplasmosis therapies can be stopped if Cluster of Differenciation 4 (CD4) count remains very high for at least three to six months. Side effects of most drugs can be more severe in people with HIV/AIDS. Toxoplasmosis is also an important veterinary infection in ovines, bovines, caprines and pigs, and is responsible for spontaneous abortion. Efficient drugs with low side effects, acting on cyst forms and usable both for human therapies and preventions in cattle farms are therefore highly needed.

The search for effective therapeutics for toxoplasmosis received much less interest and only a few examples are reported. The main target is dihydrofolate reductase (DHFR) and some inhibitors with high ligand efficiency were reported; for example starting from a QSAR analysis some authors obtained the synthesis of new 6-fluoroquinolones as potential active against *Toxoplasma gondii* (Anquetin G., et al., Eur. J. Med. Chem., 2006, 41, 1478-1493).

Very recently others reported the SAR studies based on trimethoprim and methotrexate analogues. Starting from this 1960-drug they obtained high *in vitro* inhibitory activities toward a homology model of DHFR.

In the case of babesiosis, infection is similar to malaria. Transmission of *Babesia* parasites is due to bites by ticks. Wild animals (rodents and bovids) are final or intermediary parasitic reservoirs. Some *Babesia,* such as *Babesia microti* and *Babesia divergens* can infect humans. Babesiosis is reported in various domestic species, including bovids, equids, and dogs with a large spectrum of symptom, from non-severe cases to lethality. Untreated canine babesiosis is always lethal. Besides a partially efficient vaccine, no drug treatment is currently available to fight against all *Babesia* species. Human treatments include the use of quinine combined with clindamycin, or hydroxychloroquine. For veterinary purposes, bovine and equine babesiosis caused by only two species can be mainly treated, using imidocarb dipropionate.

In the case of neosporosis, data are fragmentary and poor. *Neospora* is a parasite closely related to *Toxoplasma,* capable of infecting virtually any mammal. Dog is the final host were sexual reproduction occurs. Cattles can be infected due to proximity with infected wild animals. Drugs used to treat toxoplasmosis are used to treat neosporosis.

In the case of coccidiosis, tens of species of the *Eimeria* genus can cause infections of the gastro-intestinal system of birds and mammals. Coccidiosis is a very frequent plague in bovids, ovids, birds, rodents, etc. Massive poultry breeding requires prophylaxis against avian coccidiosis, particularly against *Eimeria necatrix* or *Eimeria tenella* that cause severe infections. Drug treatments include the prescription of antibiotics or non-antibiotic molecules such as decoquinate, a molecule of the hydroxyquinoline family.

Glutamine amidotransferase/aminodeoxychorismate synthase (GAT-ADCS) is known as a bifunctional enzyme of the folate pathway, involved in the synthesis of p-aminobenzoate (pABA). The GAT domain releases NH₃ from glutamine and the ADCS domain uses NH₃ to aminate chorismate. GAT-ADCS is found in all eukaryotic organisms autonomous for folate synthesis, such as parasites of the *Apicomplexa phylum* or plants.

The pABA moiety is synthesized in two steps from chorismate. In a first step, chorismate is aminated to form 4-amino-4-deoxychorismate (ADC), a reaction catalyzed by GAT-ADCS, and then ADC is aromatized with loss of pyruvate to form pABA, a reaction catalyzed by aminodeoxychorismate lyase (ADCL). The ADC synthase (ADCS) used to catalyse the amination of chorismate is an enzyme that belongs to the group of the chorismate-utilizing enzymes, which also contains salicylate synthase, isochorismate synthase and anthranilate synthase (K. T. Ziebart et al., Biochemistry 49 (2010) 2851-2859; O. Kerbarh et al., Biochem. Soc. Trans. 33 (2005) 763-766). ADCS is found either as a monofunctional or bifunctional enzyme, the protein bearing in the latter case a glutamine amidotransferase (GAT) activity. Searches for inhibitors for pABA synthesis were only done with prokaryotic systems and the monofunctional form of the enzyme. They involved docking studies and design of chorismate analogous compounds (E. M. Bulloch et al., J. Am. Chem. Soc. 126 (2004) 9912-9913; M. C. Kozlowski et al., J. Am. Chem. Soc. 117 (1995) 2128-2140; R. J. Payne et al., Org. Biomol. Chem. 7 (2009) 2421-2429; R. J. Payne et al., Org. Biomol. Chem. 8 (2010) 3534-3542), combinatorial chemistry approaches associating potential inhibitors with a chorismate-like moiety that directed the molecule to the active site (K. T. Ziebart et al., J. Med. Chem. 53 (2010) 3718-3729; S. Dixon et al., J. Med. Chem. 49 (2006) 7413-7426), and a specific screening of a microorganism extract collection using growth inhibition of test bacteria as a marker of activity (J. Riedlinger et al., J. Antibiot (Tokyo) 57 (2004) 271-279; S. Keller et al., Angew. Chem. Int. Edit. 46 (2007) 8284-8286). Several compounds were identified by these different methods but appeared to be relatively weak inhibitors of ADCS (E. M. Bulloch et al., J. Am. Chem. Soc. 126 (2004) 9912-9913; M. C. Kozlowski et al., J. Am. Chem. Soc. 117 (1995) 2128-2140; R. J. Payne et al., Org. Biomol. Chem. 7 (2009) 2421-2429; K. T. Ziebart et al., J. Med. Chem. 53 (2010) 3718-3729). The most potent inhibitor of ADCS reported to date is an analogue of chorismate, i.e. the 2-hydroxy-4-amino-4-deoxy chorismate, exhibiting a *Kᵢ* value of 38 µM with the purified enzyme (M. C. Kozlowski et al., J. Am. Chem. Soc. 117 (1995) 2128-2140). But the *in vivo* effects of these ADCS inhibitors were not investigated.

The equation describing pABA synthesis is as follows:

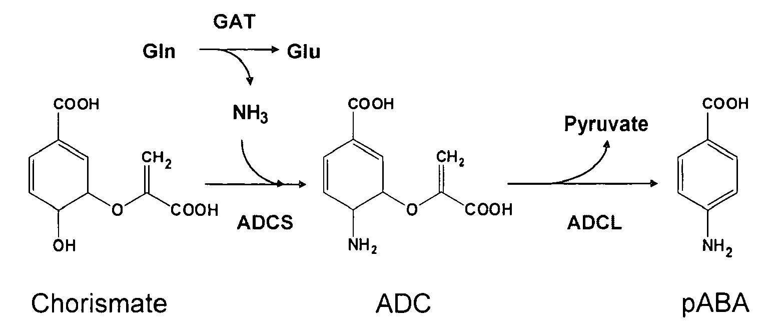

The Inventors have now surprisingly found that a novel class of compounds of general formula (I) was able to inhibit the bifunctional enzyme GAT-ADCS with an apparent inhibitory constant (*Kᵢ*) of about 10 µM. The growth of *Arabidopsis* seedlings was inhibited by such compounds, and the addition of pABA in the external medium restored growth activity. The Inventors also observed that the compounds of formula (I) are efficient to block the invasion and proliferation processes of *Toxoplasma gondii* in human fibroblasts. Their efficiency was greater than that of a well-known sulfonamide compound, i.e. sulfadiazine, used in standard treatment of toxoplasmosis and targeting dihydropteroate synthase. These observations validate the use of GAT-ADCS as a target for anti-parasitic medicaments, and indicate that the compounds of the invention as new inhibitors having antifolate activity could be an alternative to sulfonamides in therapies against parasites of the *Apicomplexa phylum* (toxoplasmosis, malaria).

Rapidly dividing cells such as embryonic cells, tumors, bacteria and parasites rely heavily on the availability of folates. This mechanism has been exploited for the development of antifolate drugs against cancer cells and microbial or parasitic infections. Biosynthesis of folate is mainly inhibited by two groups of compounds:
i) sulfonamides which are competitive inhibitors of dihydropteroate synthase (DHPS), an enzyme involved in the condensation of the pABA moiety with the pterin ring to form dihydropteroate, and
ii) inhibitors of dihydrofolate reductase (DHFR), an enzyme involved in the reduction of dihydrofolate into tetrahydrofolate, the active form of folate.

Inhibitors of DHFR are commonly used as therapeutic agents for cancer (J. R. Bertino, Best Practice & Research Clinical Haematology 22 (2009) 577-582) whereas a combination of these two types of inhibitors are most often used in clinical treatments against parasites from the *Apicomplexa phylum* such as *Plasmodium falciparum* or *Toxoplasma gondii* (A. Nzila, Drug Discov. Today 11 (2006) 939-944; P. Wang et al., Mol. Microbiol. 51 (2004) 1425-1438).

The Inventors have developed the subject of the present invention in order to treat some specific infections such as malaria or numerous babesiosis, or some parasitic stages that are currently resistant to drugs, such as toxoplasmic cysts.

A first subject of the invention is therefore a compound of general formula (I) below: 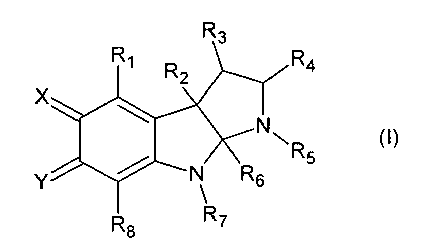 or a pharmaceutically acceptable salt thereof, wherein:
- X and Y, identical or different, are selected from O, S and NR, wherein R = H or an optionally substituted alkyl radical containing 1 to 12 carbon atoms,
- R₁, R₂, R₃, R₄, R₅, R₆, R₇ and R₈, identical or different, are selected from -H, -OH, - CH₂-OH and an optionally substituted alkyl radical containing 1 to 12 carbon atoms, for use as a medicament.

According to a preferred embodiment, X and Y are oxygen atoms.

According to another preferred embodiment, R₂, R₅ and R₇, identical or different, are selected from -CH₃, -CH₂-OH, -C₂H₅ and -C₂H₄-OH.

According to another preferred embodiment, R₁, R₃, R₄, R₆ and R₈ are hydrogen atoms.

As a particular compound of formula (I) above, we can mentioned the rubreserine responding to the following formula: 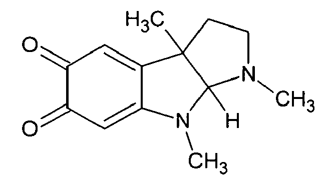 or one of its pharmaceutically acceptable salt.

Rubreserine is an oxidation product of eseroline, eseroline being itself a degradation product of physostigmine (eserine), an indole alkaloid isolated from the Calabar bean (*Physostigma venenosum*). Rubreserin according to the invention inhibits the bifunctional GAT-ADCS involved in the biosynthesis of folates (*Ki* < 10 µM). It was observed that eselorine itself has no or little effect on the enzyme activity, which indicates that the carbonyl functions (o-diquinone structure) resulting from eseroline oxidation are essential to obtain the inhibitory effect. The Inventors also observed that the incubation of rubreserine with a reducing agent such as dithiothreitol (DTT) suppressed the inhibitory effect, which confirms the importance of the quinonoid structure.

Rubreserine is not commercially available but can be easily and spontaneously obtained from buffered eseroline solutions, eseroline being commercially available. Rubreserine can be prepared as described in S. T. Yang et al., J. Pharm. Biomed. Anal 5 (1987) 383-393; B. Robinson, J. Pharm. Pharmacol. 17 (1965) 89-91; O. Poobrasert et al., J. Nat. Prod. 59 (1996) 1087-1089. In specific conditions, eseroline can also be transformed into rubreserine in the human or animal body.

Another subject matter of the invention is a compound of formula (I) for use as a medicament for the prevention and/or the treatment in human and other mammals of parasitic disease involving apicomplexan parasites, such as *Plasmodium, Babesia, Toxoplasma, Neospora, Cryptosporidium, Theileria, Sarcosystis, Eimeria* and *Gregarina,* and more preferably for the prevention and/or the treatment in human and other mammals of malaria or toxoplasmosis.

Another subject matter of the invention is a pharmaceutical composition comprising at least one compound of formula (I) as an active principle, and at least one pharmaceutically acceptable excipient.

The expression "pharmaceutically acceptable excipient" refers to any diluents, adjuvants or vehicles, such as preserving agents, fillers, disintegrating agents, wetting agents, emulsifying agents, suspending agents, solvents, dispersion media, coatings, antibacterial and antifungal agents, isotonic and absorption delaying agents and the like.

The pharmaceutical composition of the present invention may be administered by any suitable route, for example, by oral, buccal, inhalation, sublingual, nasal, percutaneous, i.e. transdermal or parenteral (including intravenous, intramuscular, subcutaneous and intracoronary) administration. Therefore, the pharmaceutical composition of the invention can be provided in various forms, such as in the form of hard gelatin capsules, of capsules, of compressed tablets, of suspensions to be taken orally, of lozenges or of injectable solutions or in any other form appropriate to the method of administration.

The pharmaceutical composition according to the invention includes those wherein a compound of formula (I) is administered in an effective amount to achieve its intended purpose. Determination of the effective amounts is well within the capability of those skilled in the art.

A "therapeutically effective dose" refers to that amount of compound of formula (I) which results in achieving the desired effect. Toxicity and therapeutic efficacy of compound of formula (I) can be easily determined by standard pharmaceutical procedures in cell cultures or experimental animals, i.e. for determining the LD₅₀ (the dose lethal to 50% of the population) and the ED₅₀ (the dose therapeutically effective in 50% of the population). The dose ratio between toxic and therapeutic effects is the therapeutic index, which is expressed as the ratio between LD₅₀ and ED₅₀. The data obtained from such data can be used in formulating range of dosage for use in humans. The dosage of compound of formula (I) preferably lies within a range of circulating concentrations that include the ED₅₀ with little or no toxicity. The dosage can vary within this range depending upon the dosage form employed, and the route of administration.

In the particular case of rubreserine, it was observed that the intraerythrocytic growth of *Apicomplexa Phylum* was strongly inhibited by said compound, with an IC₅₀ of around 1.2 µM for *Plasmodium falciparum* (3D7 strain), showing that rubreserine exhibited anti-malarial properties. It was also observed that the effect of rubreserine was dose-dependent for *Toxoplasma gondii* (RH-YFP2 strain), the number of growth of intracellular parasites being reduced two times in the presence of 20 µM of rubreserine. The presence of pABA or 5-formyltetrahydrofolate (5-FTHF) significantly reversed the inhibition, indicating that inhibition of pABA biosynthesis, and thus folate biosynthesis, was involved in this inhibitory process.

The exact formulation, route of administration, and dosage can be chosen by the individual physician in view of the patient's conditions. Dosage amount and interval of administration can be adjusted individually to provide plasma levels of compound of formula (I) which are sufficient to maintain the preventive or therapeutic effects.

The amount of pharmaceutical composition administered will therefore depend on the subject being treated, on the subject's weight, the severity of the affliction and the manner of administration.

For human and other mammal use, the compounds of formula (I) can be administered alone, but they are preferably administered in admixture with at least one pharmaceutically acceptable carrier, the nature of which will depend on the intended route of administration and the presentation form. Pharmaceutical composition for use according to the present invention thus can be formulated in a conventional manner using one or more physiologically acceptable carriers comprising one or more excipient(s) and/or auxiliary(ies) that facilitate processing of the compounds of formula (I) into preparations which can be used pharmaceutically. Amongst the excipients and auxiliaries which can be used in the pharmaceutical composition according to the invention, one can mention anti-agglomerating agents, preservatives agents, dyes, vitamins, inorganic salts, taste-modifying agents, smoothing agents, coating agents, isolating agents, stabilizing agents, wetting agents, anti-caking agents, dispersing agents, emulsifying agents, aromas, penetrating agents, solubilizing agents, etc., mixtures thereof and generally any excipient conventionally used in the pharmaceutical industry.

By way of example, when the pharmaceutical composition is administered orally, the carrier may comprise one or several excipients such as talc, lactose, starch or modified starches, cellulose or cellulose derivatives, polyethylene glycols, acrylic acid polymers, gelatin, magnesium stearate, animal or vegetal fats of natural or synthetic origin, paraffin derivatives, glycols, etc.

In addition to the at least one compound of formula (I), the pharmaceutical composition may also comprises one or more additional antiparasitic active principles, for example anti-malarial drugs such as for example chloroquine, quinacrine, primaquine, artemisinin, atovaquone and pyrimethamine.

For general information about the formulation and administration of pharmaceutical compositions, one can obviously refer to the book "Remington's Pharmaceutical Sciences", last edition. Of course, a person skilled in the art will take care on this occasion that the excipient(s) and/or auxiliary(ies) optionally used are compatible with the intrinsic properties attached to the pharmaceutical composition in accordance with the invention.

These pharmaceutical compositions can be manufactured in a conventional manner, i.e. by conventional mixing, dissolving, granulating, dragee-making, emulsifying, encapsulating, entrapping or lyophilizing processes. Proper formulation is dependent upon the route of administration chosen.

Finally, the invention also concerns the use of a compound of general formula (I) below: 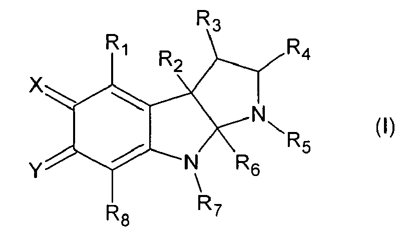 or a plant compliant salt thereof,
wherein X, Y, R₁, R₂, R₃, R₄, R₅, R₆, R₇ and R₈ are as defined above,
as an herbicide and/or an algaecide, and herbicide and/or algaecide compositions comprising at least one compound of formula (I) suitable for use against harmful plants in crops or useful plants.

More preferably, the compound of formula (I) used as an herbicide and/or an algaecide, and in herbicide and/or algaecide compositions, is the rubreserine responding to the following formula: 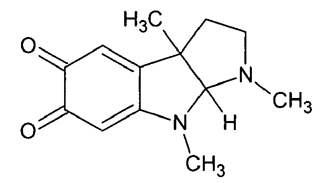 or one of its plant compliant salt.

In addition to the above provisions, the invention also comprises other provisions which will become clear from the description which follows, which refers to examples illustrating the antiparasitic activity of a compound of formula (I), *i.e.* the rubreserine, and also to the attached drawings in which:

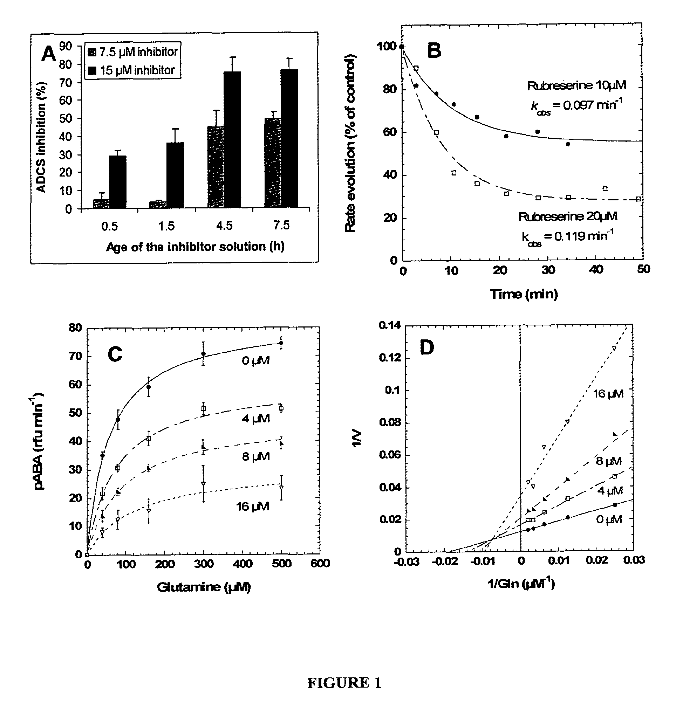

- **Figure 1** shows the effect of rubreserine on GAT-ADCS kinetics. **Figure 1A** represents the time-dependent efficiency of the eseroline (triplicate experiment ± Standard Deviation (SD)). **Figure 1B** represents the time-dependent decrease of the GAT activity following the addition of 10 and 20 µM of rubreserine. Curves were fitted using the KaleidaGraph software (Sinergy Software) and a decreasing exponential equation. **Figure 1C** shows the effect of varying Glutamine upon pABA production in the presence of 100 µM of chorismate and 0, 4, 8 and 16 µM of rubreserine. GAT-ADCS was first incubated with the various concentrations of rubreserine for 20 minutes, and then the kinetic was started by the addition of Glutamine, chorismate and ADCL (each point is the average of three independent measures ± SD). Curves were fitted using the KaleidaGraph software (Sinergy Software) and the hyperbolic equation of Michaelis-Menten. **Figure 1D** represents a reverse plot of **Figure 1C****.** Maximal rates of the recombinant enzyme were 130 ± 30 nmol.min⁻¹.mg⁻¹. Curves were fitted using the KaleidaGraph software (Sinergy Software) and a linear regression,

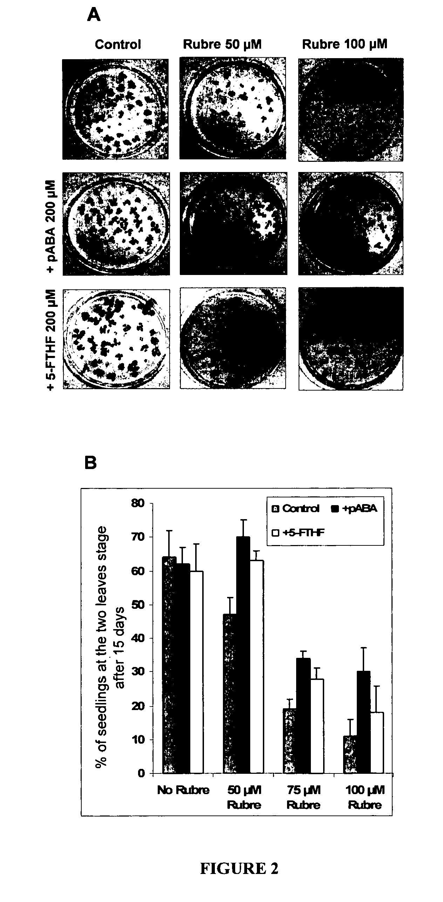

- **Figure 2** shows the effect of rubreserine (Rubre) on the development of Arabidopsis seedlings. **Figure 2A** are representative experiments showing two weeks old seedlings grown in agar plates without (control) or with 50 or 100 µM of rubreserine and in the presence or absence of 200 µM pABA or 200 µM 5-FTHF. **Figure 2B** shows histogram representing the estimated number of seedlings at the rosette stage in the various situations shown in **Figure 2A****.** Results represent the average of four independent measures ± SD,

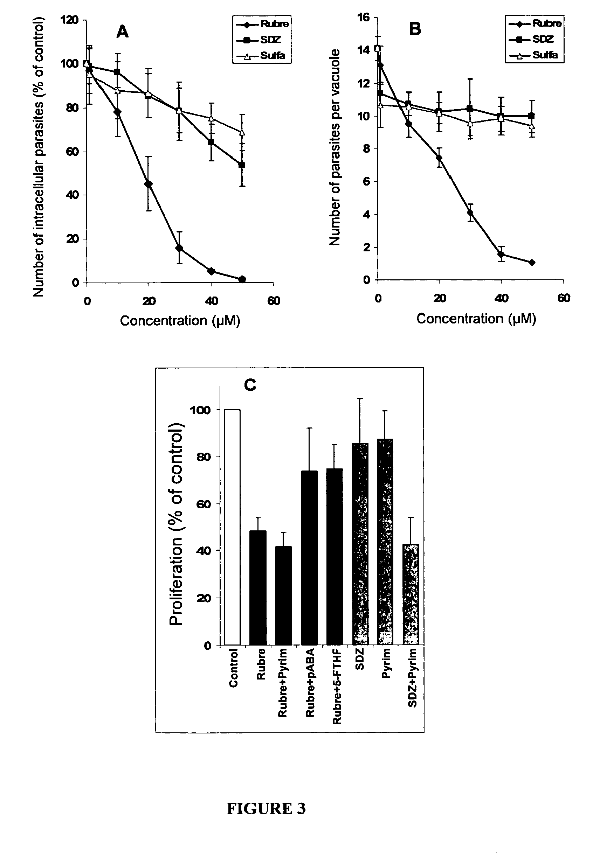

- **Figure 3** shows the effect of rubreserine (Rubre) on the invasion and proliferation processes of *Toxoplasma gondii,* and comparison with sulfanilamide (Sulfa) and sulfadiazine (SDZ). **Figure 3A** shows the invasion of human fibroblast. The parasites were first incubated with the various drugs for 5 hours, and then put in contact with the human cells for 15 minutes. The number of intracellular parasites is expressed for each experiment as a % of the number recorded in non-treated cells. **Figure 3B** shows the intracellular growth of the parasites. Intracellular proliferation was estimated 24 hours after the invasion process and expressed as the number of parasites present in the parasitophorous vacuoles. In each experiment, 200 vacuoles were counted for each drug concentration. Invasion and proliferation experiments were made in triplicate ± SD. **Figure 3C** shows the effect of rubreserine in combination with other compounds on the intracellular proliferation of the parasite. The concentrations used were: 20 µM rubreserine (Rubre), 0.4 µM pyrimethamine (Pyrim), 50 µM sulfadiazine (SDZ), 100 µM pABA and 100 µM 5-FTHF. The reversed effect of pABA and 5-FTHF illustrates that a blocking of folate biosynthesis was part of the inhibitory process,

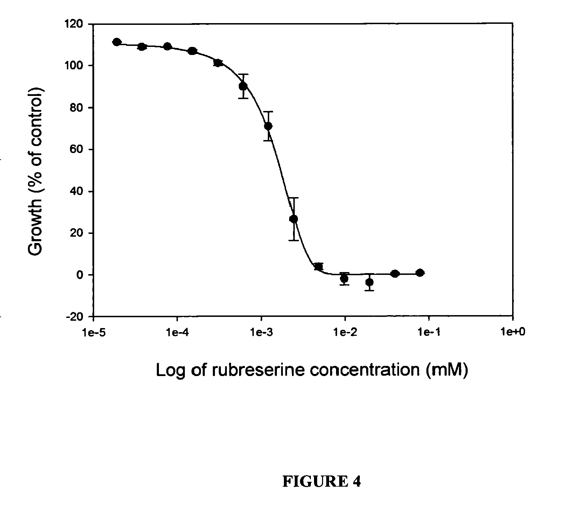

- **Figure 4** shows the effect of rubreserine on the growth of *Plasmodium falciparum* (duplicate experiments).

### EXAMPLES:

### Preparation of rubreserine:

Eseroline can be spontaneously transformed into rubreserine in slightly alkalinesolution such as bicarbonate Ringer's solution (S. Ellis et al., Pharmacol., Exp. Ther. 79 (1943), 309-319). In the present study, rubreserine was prepared from commercially available (-) eseroline fumarate salt (Sigma-Aldrich, ref. E100). Stock solutions of eseroline (10 mM) were made in 50 mM phosphate buffer (pH = 8) for *Arabidopsis* and *Toxoplasma* experiments, 50 mM Tris buffer (pH = 8) for GAT-ADCS experiments, and 1 × PBS buffer (pH = 7.5) for *Plasmodium* experiments. Under these conditions, eseroline is spontaneously oxidized into rubreserine. The formation of rubreserine was controlled through the appearance of a characteristic peak of absorption at 473 nm, a process completed in about 6 hours. Rubreserine formation was confirmed by the appearance of two resonance peaks in the carbonyl region of the ¹³C-NMR spectrum. It was also verified by HPLC analysis that the conversion was > 90% in these conditions. These stock solutions were stored at 4°C for 48 hours or at -20°C for one week. They were serially diluted before use. Chemical structures of eseroline and rubreserine are given below:

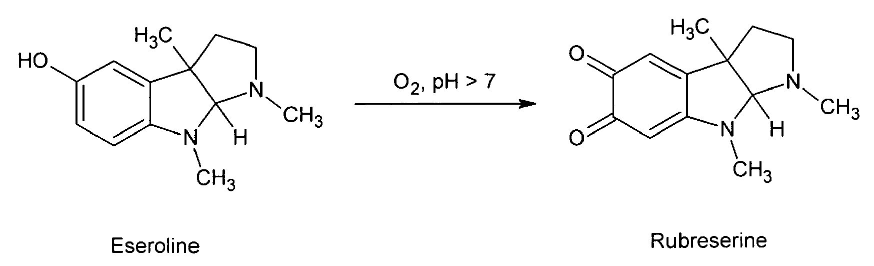

### Materials and Methods:

### Arabidopsis thaliana cultures.

*Arabidopsis thaliana* (ecotype Columbia) seedlings were grown on plates containing Murashige and Skoog medium, 15% agar, plus the various molecules to be tested. Seeds were first sterilized by soaking for 15 minutes in a solution containing 0,095% Tween^{®} 20 and 0,57% sodium hypochlorite before to be laid on the agar medium. The plates were conserved in the dark at 4°C for 48 hours, and then transferred in a green house (20°C, 80% humidity, 150 µE.m⁻².s⁻¹, 12 hours light period). The number of seedlings at the two leaf stage (rosette stage) was counted after 2 weeks.

*Arabidopsis thaliana* (ecotype Columbia) cell suspension cultures were grown under continuous light (40 µE.m⁻².s⁻¹) at 23°C with rotary agitation at 125 rpm in Gamborg's B5 medium supplemented with 1 µM 2-naphtalene acetic acid and 1.5% (w/v) sucrose. Cells were sub-cultured every 7 days in fresh medium. For measurements of metabolites, cells were collected after 7 days of treatment, rapidly washed with distilled water, weighted, frozen in liquid nitrogen and stored at -80°C for later analyses.

### Toxoplasma gondii cultures.

*Toxoplasma gondii* tachyzoites (RH-YFP2 strain) were propagated in human foreskin fibroblasts (HFFs). Human cells were grown in DMEM supplemented with 100 U/mL penicillin, 100 mg/mL streptomycin, 2 mM glutamine, and 10% FBS (Gibco). To obtain extracellular parasites, host cells were forced through a 26.5 gauge needle, and then the released parasites were purified by filtration through a 3 µm polycarbonate membrane before use.

For the invasion assay, RH-YFP2 parasites were incubated for 5 hours with the different drugs. Then 10⁶ parasites/well were centrifuged for 30 seconds at 1300 rpm on HFF monolayers to synchronize invasion and wells were incubated for 15 minutes in a water bath at 37°C. Wells were washed three times with cold PBS to eliminate extracellular parasites and cells were fixed in 2.5% formaldehyde/PBS for 30 minutes and stored in PBS at 4°C until staining. To distinguish intracellular from extracellular parasites, cells were incubated with the primary antibody mAb Tg05-54 to detect the major *Toxoplasma* surface protein SAG1 (TgSAG1), then with Texas Red-conjugated goat anti-mouse secondary antibody (Molecular Probes). Intracellular parasites exhibit a faint red colour and can easily be distinguished from extracellular parasites which are bright red. Nuclei were stained with Hoechst 33258 (Molecular Probes). To determine the number of intracellular parasites, 12 randomly selected fields were counted per well and per experiment with a Zeiss Axioplan 2 microscope equipped for epifluorescence and phase-contrast. Invasion was expressed as % of the number of intracellular parasites recorded in non-treated cells.

For the proliferation assay, 10⁵ parasites/well were centrifuged on HFF monolayers for 30 seconds at 1300 rpm and incubated for 15 minutes in a water bath at 37°C for invasion. Wells were then washed three times with PBS to eliminate extracellular parasites and drugs were added. After 24 hours at 37°C in a humidified atmosphere containing 5% CO₂, cells were fixed as described above. The number of parasites from up to 200 individual vacuoles was counted for each drug concentration.

### Plasmodium falciparum cultures.

The 3D7 strain of *Plasmodium falciparum* was maintained in O+ blood. A rubreserine 10 mM stock concentration was made up in 1 × PBS pH = 7.5. This stock solution was then diluted serially to obtain the growth curve. Growth inhibition was measured after 96 hours by using an *in vitro* SYBR Green 1-based fluorescence growth assay, as previously described (T. N. Bennett et al., Antimicrob. Agents Chemoter. (2004) 48 1807-1810; M. Smilkstein et al., Antimicrob. Agents Chemoter. (2004) 18 1803-1806).

### Expression and purification of the recombinant AtGAT-ADCS and EcADCL:

Both proteins were expressed and purified as described in the publication of D. Camara et al., Archives of Biochemistry and Biophysics 505 (2011) 83-90.

### Determination of GAT-ADCS activities:

GAT-ADCS activity can be determined by measuring the production of either glutamate or pABA. Standard assays contained 100 mM Tris/HCl (pH 8.0), 5 mM MgCl₂, 5% V/V glycerol, 0 to 0.5 mM L-glutamine, 0.1 mM chorismate, 9 yg ml⁻¹ (90 nM) of the recombinant *Arabidopsis thaliana* GAT-ADCS and either 1 mM NAD and an excess of glutamate deshydrogenase (GDH) for glutamate determination, or an excess of *Escherichia coli* ADCL (20 µg ml⁻¹, 600 nM) for pABA determination. Reactions (final volume 80 µl) were run in the wells of a 96 well microplate (Greiner) and changes in fluorescence were continuously monitored with a microplate scanning spectrophotometer Safir 2 (Tecan). The glutamate production was followed by the fluorescence emission of NADH at 450 nm (excitation 340 nm). The pABA production was directly followed by the increase of the fluorescence emission at 340 nm (excitation 290 nm).

### High throughput screening:

The equipment involved an automated screening station (Tecan Genesis 2000, Lyon, France), including a microplate reader. The platform is controlled by software for driving of serial devices and assays scheduling (Tecan Gemini 4.2 and FACTS 4.82 software). The library of compounds was purchased from The Prestwick Chemical Library^{®}. This library contains 1200 molecules, 100% being marketed drugs, supplied at a 5 mM concentration in DMSO. The final concentration used in the enzymatic assay was 100 µM, and it was verified in separate control experiments that 2% DMSO had no effect on the activity. The enzymatic test used to determine GAT-ADCS activity in primary and secondary screenings was based on glutamate determination, as described above. After optimization of the assay, the Z' factor was comprised between 0.68 and 0.84.

### Results:

### GAT-ADCS inhibition:

Eseroline was identified by high throughput screening of The Prestwick Chemical Library^{®}. When GAT-ADCS was incubated with a fresh solution of eseroline, the rate of inhibition increased with the age of the solution (see **Figure 1A****),** indicating that inhibition was linked to the pH-dependent transformation of eseroline into rubreserine (see Material and Methods). **Figure 1A** also illustrates that eseroline has to be transformed into rubreserine before becoming an active compound. The rate constant inhibition for rubreserine *kᵢ* was rather low and a 20 minutes period of incubation was required to obtain maximal inhibition (see **Figure 1B****).** Rubreserine decreased the *Vₘ* for pABA production and increased the *Kₘ* for glutamine, indicating a mixed-type inhibition (see **Figures 1C and 1D****).** The kinetic constants *Kᵢ* and *αKᵢ* calculated from such pattern of curves were estimated to be respectively 4 and 9 µM. In control experiments it was verified that rubreserine had no effect on ADCL and that inhibition is solely imputable to a decrease of GAT-ADCS activity.

### Effect of rubreserine on Arabidopsis thaliana:

When *Arabidopsis thaliana* seedlings were grown on agar plates in the presence of various concentrations of rubreserine, a dose-dependent growth inhibition was observed (see **Figures 2A and 2B****).** When the agar plates were supplemented with 200 µM pABA together with rubreserine, the inhibition was suppressed or largely reduced (see **Figure 2B****).** This indicated that growth in the presence of rubreserine was limited by pABA synthesis. Because pABA is required for folate biosynthesis, we also measured the potential impact of 5-formyl tetrahydrofolate on rubreserine-treated plants (see **Figure 2B****).** This folate derivative was also able to revert the inhibitory effect of rubreserine, but to a lower extent than pABA.

### Effect of rubreserine on Toxoplasma gondii:

Firstly, it was verified that rubreserine concentrations up to 50 µM had no effect on confluent human fibroblast cells. The effect of rubreserine on *Toxoplasma gondii* parasites was then studied in two situations:
i) in invasion of human fibroblasts, and
ii) in intracellular development of the parasites.

As shown in **Figure 3A****,** invasion of human cells strongly decreased when the parasites have been first incubated for 5 hours with various rubreserine concentrations, and then put in contact with the human cells for 15 minutes. This effect was dose-dependent, the number of intracellular parasites being reduced two times in the presence of 20 µM rubreserine. Likewise, intracellular proliferation was estimated 24 hours after the invasion process and expressed as the number of parasites present in the parasitophorous vacuoles. In each experiment, 200 vacuoles were counted for each drug concentration. When parasited cells were placed in the presence of the inhibitor, the number of parasites in the parasitophorous vacuoles decreased, which is representative of a slowing down of the parasite intracellular division (see **Figure 3B****).** This effect was reversed by pABA as shown in **Figure 3C****.** Also shown in **Figures 3A and 3B****,** is the comparison between rubreserine, and two sulfonamide drugs, sulfanilamide and sulfadiazine. Indeed, sulfadiazine is widely used in standard treatment to cure toxoplasmosis. Interestingly, rubreserine appeared more efficient than the two sulfa- drugs on either the invasion of human fibroblasts or the intracellular replication of the parasites. From these curves, an IC₅₀ for rubreserine of about 20 µM can be calculated, a value lower than the IC₅₀ for sulfadiazine estimated from this study (> 50 µM) or generally reported in the literature (> 30 µM) (P. Meneceur, Antimicrob. Agents Chemother. 52 (2008) 1269-1277). The presence of 100 µM pABA or 100 µM 5-FTHF significantly reversed the inhibition, indicating that inhibition of pABA biosynthesis (and thus of folate biosynthesis) was involved in the inhibitory process (see **Figure 3C****).** When rubreserine 20 µM was used in combination with pyrimethamine 0.4 µM, the inhibition was increased compared to rubreserine alone. Such a combination appeared as much efficient as a mixture combining 50 µM sulfadiazine and 0.4 µM pyrimethamine (see **Figure 3C****).**

### Effect of rubreserine on Plasmodium falciparum:

Rubreserine was also tested on *Plasmodium falciparum.* As shown on **Figure 4****,** rubreserine exhibited anti-malarial properties, since the intraerythrocytic growth of the parasite was strongly inhibited by rubreserine, with an IC₅₀ of around 1.2 µM.

## Claims

1. A compound of general formula (I) below: 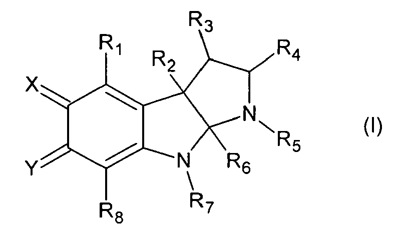 or a pharmaceutically acceptable salt thereof, wherein:
• X and Y, identical or different, are selected from O, S and NR, wherein R = H or an optionally substituted alkyl radical containing 1 to 12 carbon atoms,
• R₁, R₂, R₃, R₄, R₅, R₆, R₇ and R₈, identical or different, are selected from -H, -OH, - CH₂-OH and an optionally substituted alkyl radical containing 1 to 12 carbon atoms, for use as a medicament.

2. The compound according to Claim 1, wherein X and Y are oxygen atoms.

3. The compound according to Claim 1 or Claim 2, wherein R₂, R₅ and R₇, identical or different, are selected from -CH₃, -CH₂-OH, -C₂H₅ and -C₂H₄-OH.

4. The compound according to Claims 1 to 3, wherein R₁, R₃, R₄, R₆ and R₈ are hydrogen atoms.

5. The compound according to Claims 1 to 4, wherein said compound of formula (I) is the rubreserine responding to the following formula: 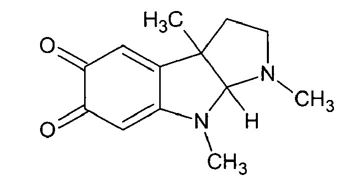 or one of its pharmaceutically acceptable salt.

6. The compound according to Claims 1 to 5, for use as a medicament for the prevention and/or the treatment of parasitic diseases involving apicomplexan parasites.

7. The compound according to Claim 6, wherein said apicomplexan parasites are selected from *Plasmodium, Babesia, Toxoplasma, Neospora, Cryptosporidium, Theileria, Sarcosystis, Eimeria* and *Gregarina.*

8. The compound according to Claims 1 to 5 for use as a medicament for the prevention and/or the treatment of malaria.

9. The compound according to Claims 1 to 5 for use as a medicament for the prevention and/or the treatment of toxoplasmosis.

10. A pharmaceutical composition comprising at least one compound of formula (I) as defined according to Claims 1 to 9 as an active principle, and at least one pharmaceutically acceptable excipient.

11. The pharmaceutical composition of Claim 10, wherein it further comprises at least one additional antiparasitic active principle.

12. The pharmaceutical composition of Claim 11, wherein said additional antiparasitic active principle is selected from chloroquine, quinacrine, primaquine, artemisinin, atovaquone and pyrimethamine.

13. Use of a compound of general formula (I) below: 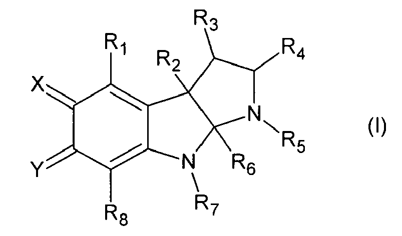 or a plant compliant salt thereof,
wherein X, Y, R₁, R₂, R₃, R₄, R₅, R₆, R₇ and R₈ are as defined according to Claims 1 to 4, as an herbicide and/or an algaecide.

14. Use according to Claim 13, wherein said compound of formula (I) is the rubreserine responding to the following formula: 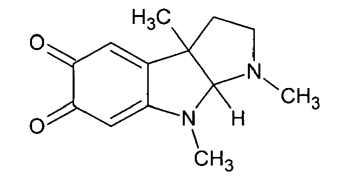 or one of its plant compliant salt.

15. An herbicide and/or algaecide composition comprising at least one compound of formula (I) as defined according to Claim 13 or Claim 14.
